Europäisches Patentamt

(19) European Patent Office

Office européen des brevets

(11) Publication number: **0 243 652**
**B1**

## (12) EUROPEAN PATENT SPECIFICATION

(45) Date of publication of the patent specification:
03.10.90

(51) Int. Cl.⁵: **C07C 51/363**, C07C 63/24

(21) Application number: 87104015.0

(22) Date of filing: 18.03.87

(54) Synthesis of high purity 5-chloroisophthaloyl chloride.

(30) Priority: 30.04.86 US 858468

(43) Date of publication of application:
04.11.87 Bulletin 87/45

(45) Publication of the grant of the patent:
03.10.90 Bulletin 90/40

(84) Designated Contracting States:
BE DE FR GB IT NL

(56) References cited:
EP-A- 0 001 252
US-A- 3 816 526
US-A- 3 869 510
US-A- 3 996 274
US-A- 4 560 794

CHEMICAL ABSTRACTS, vol. 43, no. 2, 25th
January 1949, column 1, abstract no. 609e, Columbus,
Ohio, US; N. RABJOHN: "Chlorination of isophthaloyl
and terephthaloyl chloride", & JOURNAL OF THE
AMERICAN CHEMICAL SOCIETY 1948, 70, 3518

(73) Proprietor: OCCIDENTAL CHEMICAL CORPORATION,
P.O. Box 189, Niagara Falls New York 14302(US)

(72) Inventor: Maul, James J., 15 Beaver Lane, Grand Island
New York(US)

(74) Representative: Vossius & Partner,
Siebertstrasse 4 P.O. Box 86 07 67,
D-8000 München 86(DE)

Note: Within nine months from the publication of the mention of the grant of the European patent, any person may give notice to
the European Patent Office of opposition to the European patent granted. Notice of opposition shall be filed in a written reasoned
statement. It shall not be deemed to have been filed until the opposition fee has been paid (Art. 99(1) European patent
convention).

## Description

This invention relates to an improved process for the preparation of 5-chloroisophthaloyl chloride. The product is useful as a chemical intermediate in the preparation of various products, especially agricultural chemicals, such as herbicides and the like.

US-A-3,869,510 discloses a process for the preparation of 5-chloroisophthaloyl chloride by reaction of isophthaloyl chloride with chlorine gas in the presence of molybdenum pentachloride catalyst at a temperature of about 227°C. The 5-chloroisophthaloyl chloride product is shown to be useful in the further synthesis of 3,5-dichlorobenzoyl chloride which in turn is disclosed as a useful intermediate in the further preparation of various biologically active compounds.

US-A-3,821,310 discloses the use of 5-chloroisophthaloyl chloride as an intermediate in the synthesis of 1,3-bis-(2'-hydroxy-4'-methoxy-benzoyl)-5-chlorobenzene, a product useful as a light stabilizer in polymeric materials.

Although 5-chloroisophthaloyl chloride has been known to be a useful intermediate for a number of years, the processes for its production have been found to present difficulties, especially with respect to the purity of the product. In particular, prior art processes have been found to result in the simultaneous formation of undesired isomers and over-chlorinated products, most especially the undesired isomer 4-chloroisophthaloyl chloride, and over-chlorinated products including for example, dichloroisophthaloyl chlorides. The production of isomers and over-chlorinated products results in the necessity of additional separation steps, and disposal of undesired products with a resultant economic disadvantage. The 4-chloro-isophthaloyl chloride, having a boiling point close to that of 5-chloro-isophthaloyl chloride, is particularly troublesome to separate.

It is an object of this invention to provide an improved process for the preparation of 5-chloroisophthaloyl chloride having improved selectivity, specifically with the enhancement of production of 5-chloroisophthaloyl chloride in preference to 4-chloroisophthaloyl chloride at relatively low temperatures and with minimal over-chlorination. It is a further object of this invention to provide a process whereby 5-chloroisophthaloyl chloride may be prepared at an expeditious rate while realizing the desired selectivity.

It has now been found that 5-chloroisophthaloyl chloride may be prepared in excellent yields with minimal formation of undesired isomers and over-chlorinated side products by a process comprising; reacting isophthaloyl chloride with liquid chlorine in the presence of a Lewis acid catalyst at a temperature of 50° to 100°C, and preferably 55° to 85°C under autogenous conditions. The amount of chlorine supplied to the reaction mixture should be sufficient to establish a stoichiometric excess, that is, a mole ratio of chlorine:isophthaloyl chloride of greater than 1:1 and most preferably in the range of 3:1 to 6:1. There is no critical upper limit. However, ratios in excess of 10:1 of liquid chlorine:isophthaloyl chloride, normally provide no practical advantage. The autogenous pressure resulting from temperatures in the 50°–100°C range will normally be in the range of 14 to 28 bar G (200 to 400 psig).

The process of this invention is carried out in the presence of a Lewis acid catalyst such as aluminum chloride, ferric chloride or the like. The preferred catalyst, based on effectiveness, availability and economic considerations is ferric chloride. The amount of catalyst employed may vary considerably, for example, from 0.1 percent or less to 5.0 percent or more, based on the weight of isophthaloyl chloride reactant. Typically the catalyst is employed in an amount of 0.2 percent to 2.0 percent by weight, based on the weight of isophthaloyl chloride reactant.

The following specific examples are provided to further illustrate this invention in the manner in which it may be carried out. It will be understood, however, that the specific details given in the examples have been chosen for purpose of illustration and are not to be construed as a limitation on the invention. In the examples, unless otherwise indicated, all parts and percentages are by weight and all temperatures are in degrees Celsius.

### EXAMPLE 1

A mixture of 100.4 parts of isophthaloyl chloride and 1.5 parts of ferric chloride was charged to a pressure reactor. Chlorine (107 parts) was added and the reactor was heated to 75°C and maintained thereat for six hours. During that reaction time the reactor pressure ranged from 16.8–23.2 bar G (240 to 330 psig). The reaction product was analyzed by gas chromatographic techniques and was found to contain, in mole percent, 25% isophthaloyl chloride, 70.8% 5-chloroisophthaloyl chloride, 0.49% 4-chloroisophthaloyl chloride and 2.5% dichloroisophthaloyl chloride. The analysis is consistent with a pseudo first order rate constant of $k^* = 0.25$ hour$^{-1}$.

### EXAMPLE 2

A mixture of 103 parts of isophthaloyl chloride and 4.5 parts of ferric chloride was charged to a pressure reactor. Chlorine (114 parts) was added and the reactor was heated and maintained at about 60°C for a period of about 21 hours. During that reaction time, the reactor pressure ranged from 14.0 to 21.1 bar G (200 to 300 psig). The reaction product was analyzed by gas chromatographic techniques and found to contain, in mole percent, 11.1% isophthaloyl chloride, 84.1% 5-chloroisophthaloyl chloride, 0.17% 4-chloroisophthaloyl chloride, and 4.2% dichloroisophthaloyl chloride. The analysis is consistent with a pseudo first order rate constant of $k^* = 0.105$ hours–1.

For purposes of comparison, isophthaloyl chloride was reacted with chlorine under other conditions, with the results a set forth in the following examples.

EXAMPLE 3

A mixture of 500 parts of isophthaloyl chloride and 5 parts of ferric chloride catalyst was heated and maintained at about 180°C at atmospheric pressure while chlorine gas was bubbled in over a period of about 11.3 hours. Analysis of the reaction product, by gas chromatographic techniques, indicated, in mole percent, 19.0% isophthaloyl chloride, 74.7% 5-chloroisophthaloyl chloride, 2.3% 4-chloroisophthaloyl chloride, and 3.7% dichloroisophthaloyl chloride. The analysis is consistent with a pseudo first order rate constant of $k^* = 0.37$ hours$^{-1}$.

EXAMPLE 4

A) A mixture of one hundred parts of isophthaloyl chloride and 1.0 parts of ferric chloride was heated to 95°C and maintained at that temperature for 2.3 hours. A sample of the reaction mixture was analyzed by gas chromatography and found to contain 1.0% 5-chloroisophthaloyl chloride and 99% isophthaloyl chloride starting material. The analysis is consistent with a pseudo first order rate constant of $k^* = 0.006$ hours$^{-1}$ for consumption of isophthaloyl chloride.

B) An additional 0.5 parts of ferric chloride was added to the reaction mixture and the temperature (95°C) was maintained for an additional 4.5 hours. At the end of that period, a sample of the reaction mixture was analyzed by gas chromatography and found to contain, in mole percent, 91.5% isophthaloyl chloride, 8.15% 5-chloroisophthaloyl chloride, and 0.19% 5-chloroisophthaloyl chloride. The analysis is consistent with a pseudo first order rate constant of $k^* = 0.017$ hours$^{-1}$.

C) An additional 2.0 parts of ferric chloride was added and the mixture was maintained at 95°C for an additional 17.75 hours. At the end of that period, the reaction product was analyzed by gas chromatography and found to contain, in mole percent, 12.3% isophthaloyl chloride, 84.8% 5-chloroisophthaloyl chloride, 0.71% 5-chloroisophthaloyl chloride, and 2.8% dichloroisophthaloyl chloride. The analysis is consistent with a pseudo first order rate constant of $k^* = 0.124$ hours$^{-1}$.

The advantages of the present invention are further illustrated by the data from the preceding examples as set forth in the following table.

EP 0 243 652 B1

## TABLE 1

| Example | Type of Reaction | Temperature/°C | Yield/Mole % 5-chloroisophthaloyl chloride | Isomer Ratio** 5-ClIPC/4-ClIPC | Pseudo-First Order Rate Constant $k^*$ (x100)hours$^{-1}$ |
|---|---|---|---|---|---|
| 1 | Autoclave | 75 | 70.8 | 144 | 25. |
| 2 | Autoclave | 60 | 84.1 | 495 | 10.5 |
| 3 | Atmospheric | 180 | 74.7 | 32 | 37. |
| 4 A) | Atmospheric | 95 | 1.0 | -- | 0.6 |
| 4 B) | Atmospheric | 95 | 8.15 | 43 | 1.7 |
| 4 C) | Atmospheric | 95 | 84.8 | 119 | 12.4 |

*Calculated as $k = [\ln A_0/A]/T$

$A^o$ + A are the initial and final mole fraction of isophthaloyl chloride

**5-ClIPC = 5-chloroisophthaloyl chloride

4-ClIPC = 4-chloroisophthaloyl chloride

From the table it will be seen that reaction conditions of low temperature and autogenous pressure result in unexpectedly high yields and purity, effluent utilization of chlorine, and high rate of reaction.

## Claims

1. A process for the preparation of 5-chloroisophthaloyl chloride which comprises reacting isophthaloyl chloride with liquid chlorine in the presence of a Lewis acid catalyst at a temperature of 50° to 100°C under autogenous pressure.

2. A process according to Claim 1 wherein the catalyst is ferric chloride.

3. A process according to Claim 2 wherein the catalyst is present in an amount of between 0.2 and 2.0 percent by weight based on the weight of isophthaloyl chloride reactant.

4. A process according to Claim 1 wherein the chlorine and isophthaloyl chloride reactants are provided in a mole ratio of chlorine:isophthaloyl chloride of greater than 1:1.

5. A process according to Claim 4 wherein the mole ratio of chlorine:isophthaloyl chloride is 3:1 to 6:1.

6. A process according to Claim 4 wherein the catalyst is ferric chloride.

7. A process according to Claim 1 carried out at a temperature of 50° to 100°C and under autogenous pressure of 14 to 28 bar G (200 to 400 psig).

8. A process for the preparation of 5-chloroisophthaloyl chloride which comprises reacting a mixture of isophthaloyl chloride and stoichiometric excess of liquid chlorine at a temperature of 50° to 100°C under autogenous pressure, in the presence of 0.1 to 5.0 percent by weight of ferric chloride catalyst, based on the weight of isophthaloyl chloride reactant.

9. A process according to Claim 8 wherein the temperature is 55° to 85°C.

10. A process according to Claim 9 wherein the amount of ferric chloride catalyst is 0.2 to 2.0 percent by weight, based on the weight of isophthaloyl chloride.

## Patentansprüche

1. Verfahren zur Herstellung von 5-Chlorisophthaloylchlorid, welches die Umsetzung von Isophthaloylchlorid mit flüssigem Chlor in Gegenwart eines Lewissäurekatalysators bei einer Temperatur von 50 bis 100 °C unter dem sich selbst einstellenden Druck umfaßt.

2. Verfahren nach Anspruch 1, in dem der Katalysator Eisen-III-chlorid ist.

3. Verfahren nach Anspruch 2, in dem der Katalysator in einer Menge zwischen 0,2 und 2,0 Gew.-%, bezogen auf das Isophthaloylchlorid-Reaktandengewicht, zugegen ist.

4. Verfahren nach Anspruch 1, in dem Chlor und die Isophthaloylchlorid-Reaktanden in einem Molverhältnis von Chlor:Isophthaloylchlorid, das größer als 1:1 ist, eingesetzt werden.

5. Verfahren nach Anspruch 4, in dem das Molverhältnis von Chlor zu Isophthaloylchlorid 3:1 bis 6:1 beträgt.

6. Verfahren nach Anspruch 4, in dem der Katalysator Eisen-III-chlorid ist.

7. Verfahren nach Anspruch 1, das bei einer Temperatur von 50 bis 100 °C unter dem sich selbst einstellenden Druck von 14 bis 28 bar (200 bis 400 psig) durchgeführt wird.

8. Verfahren zur Herstellung von 5-Chlorisophthaloylchlorid, welches die Umsetzung eines Gemisches von Isophthaloylchlorid und einem stöchiometrischen Überschuß flüchtigen Chlores bei einer Temperatur von 50 bis 100 °C unter dem sich selbst einstellenden Druck in Gegenwart von 0,1 bis 5,0 Gew.-% Eisen-III-chloridkatalysator, bezogen auf das Isophthaloylchlorid-Reaktandengewicht, umfaßt.

9. Verfahren nach Anspruch 8, in dem die Temperatur 55 bis 85 °C beträgt.

10. Verfahren nach Anspruch 9, in dem die Menge an Eisen-III-chloridkatalysator 0,2 bis 2,0 Gew.-%, bezogen auf das Isophthaloylchloridgewicht, beträgt.

## Revendications

1. Procédé de préparation du chlorure de 5-chloroisophtaloyle, qui comprend la réaction du chlorure d'isophtaloyle avec le chlore liquide en présence d'un acide de Lewis catalytique à une température de 50° à 100°C sous la pression spontanée.

2. Procédé suivant la revendication 1, dans lequel le catalyseur est le chlorure ferrique.

3. Procédé suivant la revendication 2, dans lequel le catalyseur est présent en une quantité entre 0,2 et 2,0% en poids, sur la base du poids du réactant chlorure d'isophtaloyle.

4. Procédé suivant la revendication 1, dans lequel les réactants chlore et chlorure d'isophtaloyle sont pris dans un rapport molaire chlore:chlorure d'isophtaloyle supérieur à 1:1.

5. Procédé suivant la revendication 4, dans lequel le rapport molaire chlore:chlorure d'isophtaloyle est de 3:1 à 6:1.

6. Procédé suivant la revendication 4, dans lequel le catalyseur est le chlorure ferrique.

7. Procédé suivant la revendication 1, exécuté à une température de 50° à 100°C et sous la pression spontanée de 14 à 28 bars M (200 à 400 livres par pouce carré au manomètre).

8. Procédé de préparation du chlorure de 5-chloroisophtaloyle, qui comprend la réaction d'un mélange de chlorure d'isophtaloyle et d'un excès stoechiométrique de chlore liquide à une température de 50° à

5

100°C sous la pression spontanée, en présence de 0,1 à 5,0% en poids de chlorure ferrique catalytique, sur la base du poids du réactant chlorure d'isophtaloyle.

9. Procédé suivant la revendication 8, dans lequel la température est de 55 à 85°C.

10. Procédé suivant la revendication 9, dans lequel la quantité de chlorure ferrique catalytique est de 0,2 à 2,0% en poids, sur la base du poids du chlorure d'isophtaloyle.